# EUROPEAN PATENT APPLICATION

(11) **EP 4 059 428 A1**
(43) Date of publication of application: **21.09.2022**
(21) Application number: 20887963.5
(22) Date of filing: 22.10.2020
(51) Int. Cl.: A61B 5/25, A61B 5/256, A61B 5/263

(54) **BIOELECTRODE, PRODUCTION METHOD AND INSTALLATION METHOD FOR BIOELECTRODE**

(30) Priority: 14.11.2019 JP 2019206470
(71) Applicant: ASAHI FR R&D Co., Ltd., Saitama-shi, Saitama 330-0801 (JP)
(72) Inventor: MIHARA, Syo, Saitama-shi, Saitama 330-0801 (JP); TAKEOKA, Shinji, Tokyo 169-8050 (JP); NAKANISHI, Takenori, Tokyo 169-8050 (JP)
(74) Representative: dompatent von Kreisler Selting Werner - Partnerschaft von Patent- und Rechtsanwälten mbB
(86) International application number: PCT/JP2020/039765
(87) International publication number: WO 2021/095480

(57) **Abstract**

Provided is a bioelectrode in which an electrode layer can deform in association with the unevenness of the installation surface of the biological surface so as to adhere to the installation surface, and which can be easily transported and stored, a production method and an installation method for the bioelectrode.

It is a bioelectrode 10 in which a flexible electrode 12 that is to directly contact a biological surface is formed from an electrode layer 14, 14 that comprises a conductive polymer and deforms in association with an installation surface of the biological surface so as to adhere to the installation surface, and an elastomer layer that is layered on one surface side of the electrode layer 14, 14 and deforms in association with the installation surface and the electrode layer 14, 14, wherein the flexible electrode 12 is bonded to a water-permeable layer 18 that serves as a support via a water-soluble sacrificial layer 20 that comprises a water-soluble material.

## Description

### Technical Field

The invention relates to a bioelectrode installed to a biological surface and connected to a connection terminal of a biological measurement device, a production method and an installation method for the bioelectrode.

### Background Art

Since a biological measurement can contribute to the prevention of diseases, the improvement of healthy life expectancy, and the improvement of sports medicine, it is important to improve the measurement accuracy of biological measurement devices. In order to improve the accuracy of the biological measurement, it is necessary that the electrode connected to the connection terminal of the biological measurement device not only has excellent conductivity, but also adheres sufficiently to the installation surface of the biological surface and can accurately detect the potential difference of the living body. Although the conductivity of the electrode made of a metal plate is good, since it is not flexible, if it is tried to be adhered sufficiently to the installation surface of the biological surface, the pressure must be increased, and there is a concern about the effect on the living body.

A bioelectrode that comprises a conductive polymer instead of such electrode made of a metal plate has been proposed in Patent Literature 1 below. In this bioelectrode, an electrode part that is in contact with the skin of the biological surface is formed of an organic conductive polymer.

### [Prior Art Document]

### [Patent Document]

[Patent Document 1] JP2006-68024A

### Summary of Invention

### Problem to be Solved by the Invention

Although in the bioelectrode described in Patent Literature 1, the electrode part that is in contact with the skin on the biological surface is formed of an organic conductive polymer, a metal electrode is arranged on the back surface side of the electrode part that comprises an organic conductive polymer, thus the flexibility of the bioelectrode lacks and it is necessary to increase the pressure so that the electrode part is sufficiently adhered to the installation surface of the biological surface, therefore the burden on the subject still increases.

The present invention is made to solve the above problems, and an object of the present invention is to provide a bioelectrode in which an electrode layer can deform in association with the unevenness of the installation surface of the biological surface so as to adhere to the installation surface, and which can be easily transported and stored, a production method and an installation method for the bioelectrode.

### Means to Solve the Above Problems

Abioelectrode according to the present invention is made to achieve the above-described object. The bioelectrode is characterized in that a flexible electrode that is to directly contact a biological surface is formed from an electrode layer that comprises a conductive polymer and deforms in association with the unevenness of an installation surface of the biological surface so as to adhere to the installation surface, and an elastomer layer that is layered on one surface side of the electrode layer and deforms in association with the installation surface and the electrode layer, and the flexible electrode is bonded to a water-permeable layer that serves as a support via a water-soluble sacrificial layer that comprises a water-soluble material.

When the elastomer layer has a larger area than the electrode layer so that the exposed surface from the electrode layer adheres to the installation surface, a state in which the electrode layer sufficiently adheres to the installation surface of the biological surface can be maintained.

When the thickness of the elastomer layer is 5 µm or less, the thickness of the electrode layer is 0.3 µm or more and the total thickness of the elastomer layer and the electrode layer is 5.3 µm or less, the elastomer layer and the electrode layer can easily deform entirely in association with the installation surface of the biological surface so as to adhere to the installation surface.

When the conductive polymer is poly(3,4-ethylenedioxythiophene)-polystyrene sulfonate or similar compounds thereof, it can impart good conductivity and flexibility to the electrode layer.

When the elastomer layer is formed of polydimethylsiloxane, polyurethane or styrene butadiene thermoplastic elastomer, it can improve adhesion to the biological surface and the electrode layer.

When the water-permeable layer is formed of a nonwoven fabric made of cellulose or resin, or a sponge or mesh made of resin, it can have excellent strong supportability and water permeability.

When the water-soluble sacrificial layer is formed of starch, polyvinyl alcohol, polyacrylic acid or polyethylene glycol, the flexible electrode and the water-permeable layer can be securely bonded, and the flexible electrode can be reliably peeled off from the water-permeable layer by dissolving the water-soluble sacrificial layer with water penetrated through the water-soluble permeable layer.

When a conductive material is filled in a through hole that penetrates the elastomer layer, and a connector part that electrically connects the electrode layer to the external connection electrode installed to the other side of the elastomer layer is formed, the external connection electrode such as a measurement device and the electrode layer can be easily electrically connected.

The method for producing a bioelectrode according to the present invention made to achieve the above-described object is characterized in that a bioelectrode including a flexible electrode that is to directly contact a biological surface is formed by laminating a water-soluble sacrificial layer comprising a water-soluble material on one surface side of the water-permeable layer that serves as a support layer of the flexible electrode, and then laminating sequentially the elastomer layer and the electrode layer on the water-soluble sacrificial layer, wherein the electrode layer that comprises a conductive polymer and deforms in association with an installation surface of the biological surface so as to adhere to the installation surface, and the elastomer layer that is layered on one surface side of the electrode layer and deforms in association with the installation surface and the electrode layer constitute the flexible electrode.

By forming the elastomer layer in a larger area than the electrode layer so that the exposed surface from the electrode layer adheres to the installation surface, a state in which the electrode layer sufficiently adheres to the installation surface of the biological surface can be maintained.

By forming the elastomer layer having a thickness of 5 µm or less and the electrode layer having a thickness of 0.3 µm or more so that the total thickness of the elastomer layer and the electrode layer is 5.3 µm or less, the elastomer layer and the electrode layer can easily deform entirely in association with the unevenness of the installation surface of the biological surface so as to adhere to the installation surface.

By using poly(3,4-ethylenedioxythiophene)-polystyrene sulfonate as the conductive polymer, good conductivity and flexibility can be imparted to the electrode layer.

By forming the elastomer layer of polydimethylsiloxane, polyurethane or styrene/butadiene thermoplastic elastomer, adhesion to the biological surface and the electrode layer can be improved.

By using a nonwoven fabric made of cellulose or resin, or a sponge or mesh made of resin as the water-permeable layer, both excellent strong supportability and water permeability can be achieved.

By forming the water-soluble sacrificial layer of starch, polyvinyl alcohol, polyacrylic acid or polyethylene glycol, the flexible electrode and the water-permeable layer can be securely bonded, and the flexible electrode can be reliably peeled off from the water-permeable layer by dissolving the water-soluble sacrificial layer with water penetrated through the water-soluble permeable layer.

By filling the conductive material in a through hole that penetrates the elastomer layer and the electrode layer and forming a connector part that electrically connects the electrode layer to the external connection electrode installed to the other side of the elastomer layer, the external connection electrode such as a measurement device and the electrode layer can be easily electrically connected.

The method for installing a bioelectrode according to the present invention made to achieve the above object is characterized in that when the above-described bioelectrode is installed to the installation surface of the biological surface, the entire surface of the one surface side on which the electrode layer of the flexible electrode constituting the bioelectrode is formed is brought into contact with the installation surface, and the electrode layer is adhered to the installation surface while the elastomer layer and the electrode layer deform in association with the installation surface, then water is supplied to the water-permeable layer to dissolve a water-soluble material forming the water-soluble sacrificial layer with water penetrated through the water-permeable layer, and the water-permeable layer is peeled off from the flexible electrode.

The bioelectrode in which the elastomer layer is formed in a larger area than the electrode layer is used as the bioelectrode, and while the elastomer layer and the electrode layer deform in association with the installation surface, and the electrode layer is adhered to the installation surface, an exposed surface of the elastomer layer from the electrode layer is adhered to the installation surface, and thereby maintaining a state in which the electrode layer is sufficiently adhered to the installation surface of the biological surface.

### Effects of the Invention

Since the bioelectrode according to the present invention is supported by the water-permeable layer that serves as the support, it can be easily transported and stored. In addition, in the flexible electrode constituted of the elastomer layer and the electrode layer that are deformed in association with the installation surface of the biological surface and installed to the installation surface, the water-permeable layer that serves as the support can be easily peeled off by dissolving the water-soluble sacrificial layer with water supplied to the water-permeable layer, and the flexible electrode can be reliably installed to the installation surface of the biological surface. Moreover, the elastomer layer and the electrode layer constituting the flexible electrode easily deform even if the installation surface is an uneven surface, and the electrode layer is sufficiently adhered to the installation surface, so the accuracy of biological measurement can be improved without increasing the pressure.

### Brief Description of Drawings

[Fig. 1]

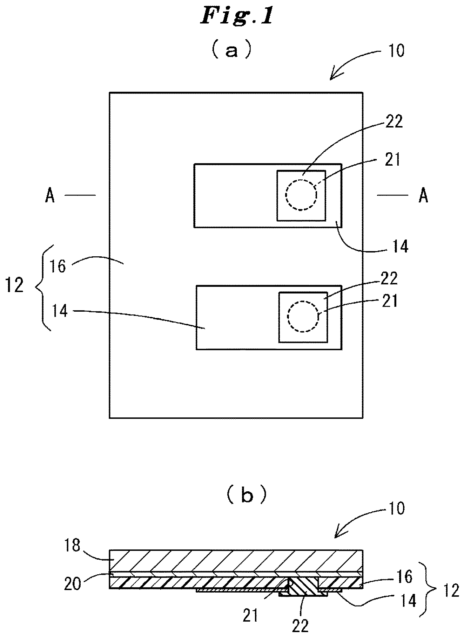

   Fig. 1 is a front view and a cross-sectional view on the A-A surface of the bioelectrode to which the present invention is applied.
[Fig. 2]

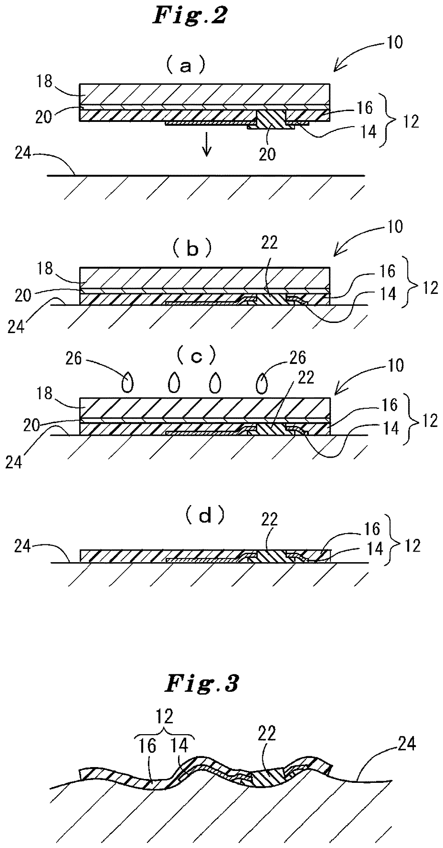

   Fig. 2 is a step diagram explaining the production method of the bioelectrode to which the present invention is applied.
[Fig. 3]
   Fig. 3 is an explanatory drawing explaining the installation method of the bioelectrode to which the present invention is applied.
[Fig. 4]

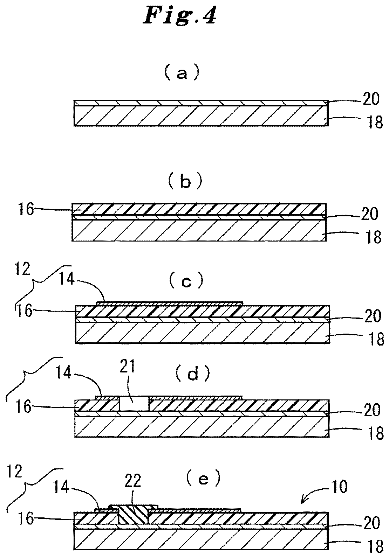

   Fig. 4 is a cross-sectional view explaining the state in which the bioelectrode to which the present invention is applied is installed to an uneven surface.
[Fig. 5]

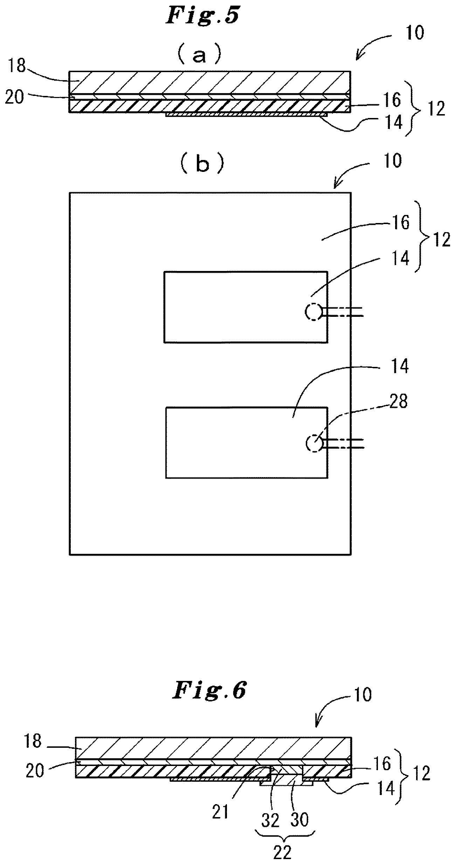

   Fig. 5 is a cross-sectional view and a front view explaining other examples of the bioelectrode to which the present invention is applied.
[Fig. 6]
   Fig. 6 is a cross-sectional view explaining other embodiments of the bioelectrode to which the present invention is applied.
[Fig. 7]

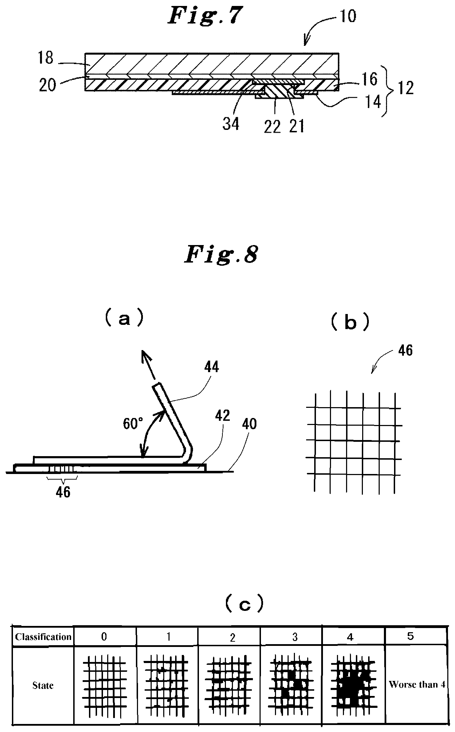

   Fig. 7 is a cross-sectional view explaining other embodiments of the bioelectrode to which the present invention is applied.
[Fig. 8]
   Fig. 8 is explanatory drawings explaining the cross-cut method for testing adhesion to the artificial skin.
[Fig. 9]

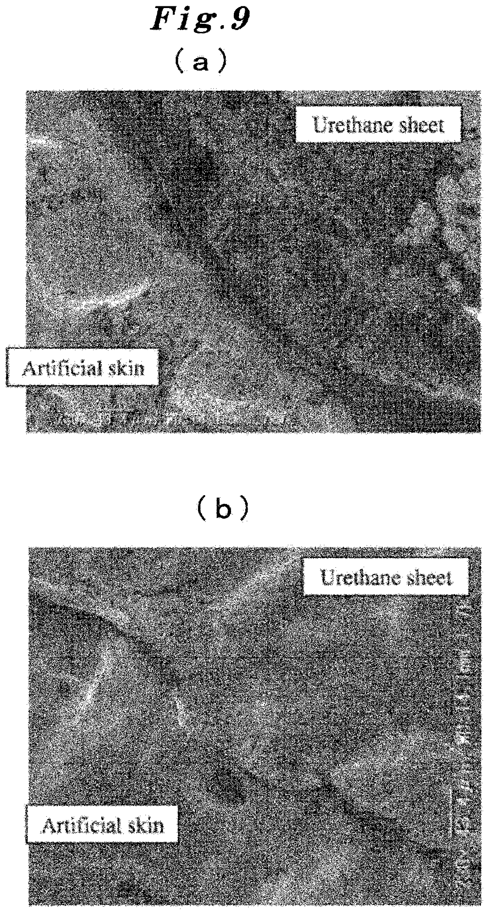

   Fig. 9 is electron micrographs of the adhesion state between the artificial skin and the urethane layer made of polyurethane as the elastomer layer.
[Fig. 10]

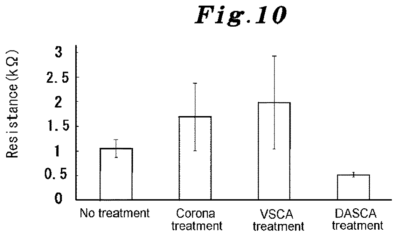

   Fig. 10 is a graph showing the relation between the surface treatment of the urethane layer and the electrical resistance value of the conductive layer.
[Fig. 11]

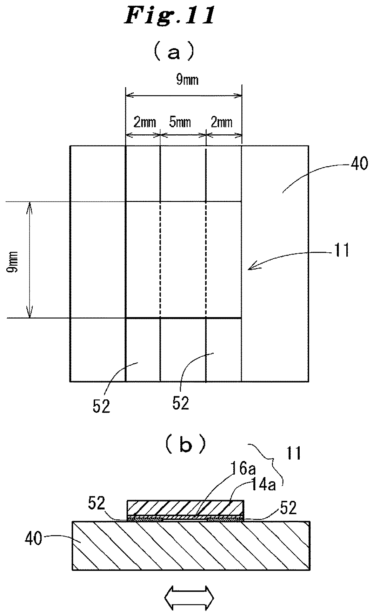

   Fig. 11 is explanatory drawings explaining the tensile durability test of the electrical resistance value of the conductive layer laminated on the urethane layer.
[Fig. 12]

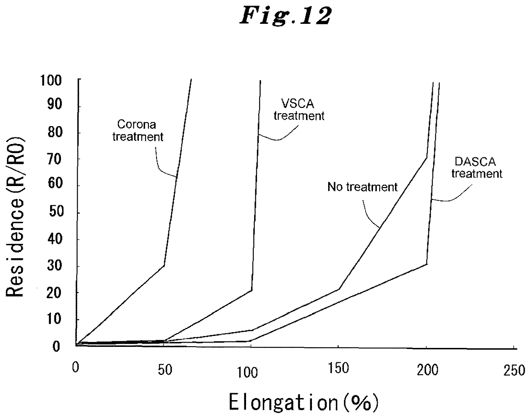

   Fig. 12 is a graph showing the tensile durability of the electrical resistance value of the conductive layer by the surface treatment of the urethane layer.
[Fig. 13]

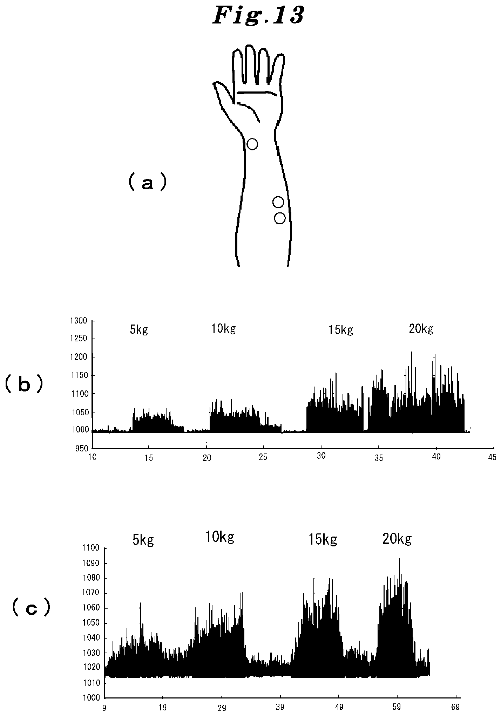

   Fig. 13 is an explanatory drawing explaining the installation position upon installing the flexible electrode of the bioelectrode to which the present invention is applied to the arm to measure the muscle potential, a measured electromyogram, and an electromyogram performed using a gel electrode commercially available as a reference electrode to measure the muscle potential.
[Fig. 14]

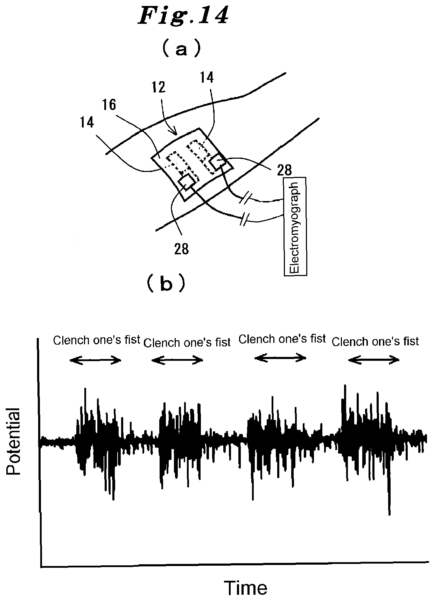

   Fig. 14 is an explanatory drawing explaining the measurement state of the muscle potential by installing the flexible electrode of the bioelectrode to which the present invention is applied to the arm and a measured electromyogram.
[Fig. 15]

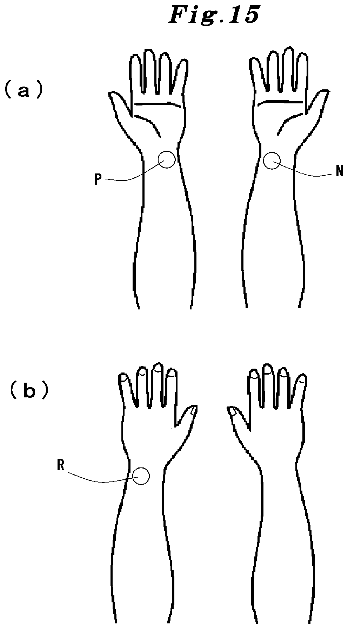

   Fig. 15 is an explanatory drawing explaining the installation position upon installing the flexible electrode of the bioelectrode to which the present invention is applied to the arm to measure the cardiac potential.
[Fig. 16]

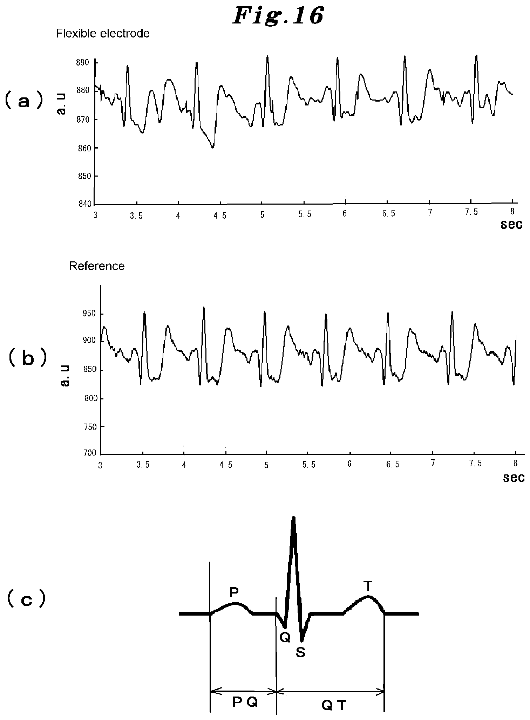

   Fig. 16 is electrocardiograms measured by installing the flexible electrode of the bioelectrode to which the present invention is applied to the arm and an explanatory drawing explaining a QT time and a QR time obtained based on this electrocardiogram.

### Embodiments to Implement the Invention

Hereinafter, the present invention will be described in detail, but the scope of the present invention is not limited to these descriptions.

A bioelectrode according to the present invention is shown in Fig. 1. Fig. 1(a) is a front view of the bioelectrode 10, and Fig. 1(b) is a cross-sectional view of Fig. 1 on the A-A surface. In the bioelectrode 10, a flexible electrode 12 consisting of rectangular electrode layers 14 and 14 and a rectangular elastomer layer 16 having a larger area than the electrode layers 14 and 14 is bonded to a water-permeable layer 18 that serves as a support of the flexible electrode 12 via a water-soluble sacrificial layer 20 comprising a water-soluble material as shown in Fig. 1.

The electrode layers 14 and 14 are formed of a conductive polymer so that they can deform in association with the installation surface of the biological surface so as to adhere to the installation surface. Although commercially available conductive polymers can be used as the conductive polymer, poly(3,4-ethylenedioxythiophene)-polystyrene sulfonate (PEDOT-PSS: hereafter, it is referred to as PEDOT-PSS) is used, and thereby imparting good conductivity and flexibility to the electrode layer 14.

The elastomer layer 16 is laminated on one surface side of the electrode layers 14 and 14, has a larger area than the electrode layers 14 and 14, and deforms in association with the installation surface of the biological surface and the electrode layers 14 and 14, so that the exposed part exposed from the electrode layers 14 and 14 adheres to the installation surface. The elastomer layer 16 is formed of polydimethylsiloxane (PDMS), polyurethane or styrene butadiene thermoplastic elastomers to improve adhesion to the biological surface and the electrode layers. In particular, when PEDOT-PSS is used as the electrode layers 14 and 14, and the elastomer layer 16 is preferably formed of polyurethane, so that both can be firmly adhered together. In particular, when the elastomer layer 16 is formed of polyurethane and the electrode layers 14 and 14 are formed of PEDOT-PSS, the elastomer layer 16 and the electrode layers 14 and 14 can be further tightly adhered together by treating the surface of the elastomer layer 16 subjected to corona discharge treatment with a diamino group-containing silane coupling agent.

The flexible electrode 12 consisting of the electrode layers 14 and 14 and the elastomer layer 16 deforms in association with the unevenness on the installation surface of the biological surface, and the state in which the electrode layers 14 and 14 are adhered to the installation surface can be maintained by the exposed surface from the electrode layers 14 and 14 of the elastomer layer 16 adhered to the installation surface. As for the thickness of the electrode layers 14 and the elastomer layer 16, when the thickness of the electrode layer 14 is 0.3 µm or more, the thickness of the elastomer layer 16 is 5 µm or less, and the total thickness of the electrode layer 14 and the elastomer layer 16 is 5.3 µm or less, preferably 5 µm or less, the state in which the electrode layers 14 and 14 are deformed in association with a fine uneven surface and adhered thereto can be reliably maintained by sufficiently adhering the exposed surface from the electrode layers 14 and 14 of the elastomer layer 16 to the skin, even with the fine uneven surface such as the skin of the human body. Furthermore, the area ratio between the electrode layers 14 and 14 and the elastomer layer 16 (the area of the electrode layers 14 and 14: the area of the elastomer layer 16) is preferably 1: 2 to 1: 18, so that the exposed surface from the electrode layers 14 and 14 of the elastomer layer 16 adheres to the skin, and the adhesion state of the electrode layers 14 and 14 adhering to the skin can be sufficiently maintained.

The flexible electrode 12 consisting of the electrode layers 14 and 14 and the elastomer layer 16 that deform in association with the shape of the installation surface is bonded to the water-permeable layer 18 that serves as a support via the water-soluble sacrificial layer 20 comprising a water-soluble material as shown in Fig. 1(b), thus the bioelectrode 10 is easily transported and stored. The water-permeable layer 18 may be any material that can permeate water, specifically a nonwoven fabric made of cellulose or resin, or a sponge or mesh made of resin. A thickness of about 0.03 to 3 mm is suitable for transportation and storage of the bioelectrode 10.

Further, the water-soluble sacrificial layer 20 may be formed of any water-soluble material that can bond the flexible electrode 12 to the water-permeable layer 18 and is soluble in water, and specifically if it is formed of starch, polyvinyl alcohol, polyacrylic acid or polyethylene glycol, the flexible electrode 12 and the water-permeable layer 18 can be reliably bonded, and water penetrated through the water-soluble permeable layer 20 dissolves it and the flexible electrode 12 can be reliably peeled off from the water-permeable layer 18.

In the flexible electrode 12 shown in Fig. 1, a connector part 22 having a through hole 21 filled with a conductive material penetrating through the elastomer layer 16 and the electrode layer 14 is formed. A part of the connector part 22 thinly and narrowly flanges and extends on the electrode layer 14. The connector part 22 electrically connects the electrode layer 14 to an external connection electrode such as a biological measurement device. Conductive rubber can be suitably used as the conductive material filled into the through hole 21. Conductive silicone rubber containing a metal filler such as silver and/or gold, copper, or fillers coated with those metals has a lower electrical resistance value than other conductive rubbers and is suitable as the conductive rubber. A combination of a conductive nonwoven fabric and a conductive adhesive can also be used as the conductive material forming the connector part 22.

The procedure for installing the bioelectrode 10 shown in Fig. 1 to the installation surface is described in Fig. 2. The installation surface 24 shown in Fig. 2 is a flat surface. First, as shown in Fig. 2(a), the electrode layer 14 of the bioelectrode 10 comes into contact with the installation surface 24, and as shown in Fig. 2(b), the entire surface of the electrode layer 14 and the exposed surface of the elastomer layer 16 exposed from the electrode layer 14 are adhered to the installation surface 24. At that time, the flanged part of the connector part 22 is extended on the electrode layer 14, but its thickness is thin and narrow, and the adhesion between the installation surface 24 and the electrode layer 14 is sufficient. Next, as shown in Fig. 2(c), water 26 is supplied to the water-permeable layer 18 of the bioelectrode 10, the water-soluble sacrificial layer 20 is dissolved with water penetrated through the water-permeable layer 18 so that the water-soluble sacrificial layer 20 is removed as shown in Fig. 2(d), and only the flexible electrode 12 consisting of the elastomer layer 16 and the electrode layer 14 can be left adhered to the installation surface 24. The end surface of the connector part 22 is exposed on the upper surface of the elastomer layer 16 of the flexible electrode 12, and an external connection electrode such as a biological measurement device is connected. Since the area of the elastomer layer 16 is larger than that of the electrode layer 14 in the flexible electrode 12, the exposed surface of the elastomer layer 16 is directly adhered to the installation surface 24, and a state in which the electrode layer 14 is sufficiently adhered to the installation surface 24 can be maintained.

Fig. 2 shows the state in which the flexible electrode 12 is installed to the flat installation surface 24, and even with the uneven installation surface 24, as shown in Fig. 3, the electrode layer 14 and the elastomer layer 16 of the flexible electrode 12 also deform in association with the uneven shape of the installation surface 24, and while the electrode layer 14 deforms in association with the installation surface 24 so as to adhere to the installation surface, the exposed surface from the electrode layer 14 of the elastomer layer 16 also deforms in association with the installation surface so as to adhere to the installation surface. Therefore, even if the installation surface 24 is an uneven surface, a state in which the electrode layer 14 of the flexible electrode 12 is sufficiently adhered to the uneven surface of the installation surface 24 can be maintained.

Fig. 4 shows the production steps of the bioelectrode 10 including the flexible electrode 12 shown in Fig. 1 to Fig. 3. First, as shown in Fig. 4(a), the water-soluble sacrificial layer 20 comprising a water-soluble material such as starch or polyvinyl alcohol is formed on one surface side of the water-permeable layer 18 formed of a water-permeable material such as a nonwoven fabric made of cellulose or resin or a sponge made of resin. Furthermore, the elastomer layer 16 consisting of an elastomer such as polydimethylsiloxane (PDMS) or polyurethane is formed on the entire surface of the water-soluble sacrificial layer 20. The elastomer layer 16 can be obtained by applying a solution in which an elastomer is dissolved in a solvent and then heat-treating at a predetermined temperature. The film thickness of the elastomer layer 16 can be controlled by adjusting the concentration of elastomer in the solution.

Next, as shown in Fig. 4(c), the electrode layer 14 comprising a conductive polymer is formed on the elastomer layer 16. The electrode layer 14 has a smaller area than that of the elastomer layer 16. The electrode layer 14 can be formed by printing a solution in which a conductive polymer is dissolved in a solvent on a mask formed with an opening in a predetermined area attached to the water-soluble sacrificial layer 20, removing the mask, and then curing at a predetermined temperature. The film thickness of the electrode layer 14 can also be controlled by adjusting the concentration of conductive polymer in the solution. Here, when the elastomer layer 16 is formed of polyurethane and the electrode layers 14 and 14 are formed of PEDOT-PSS, after corona discharge treatment is performed on the surface of the elastomer layer 16, by further treating with a diamino group-containing silane coupling agent, the elastomer layer 16 and the electrode layers 14 and 14 can be more firmly adhered together. Examples of the diamino group-containing silane coupling agent include 3-(2-aminoethyl)aminopropyltrimethoxysilane.

Thereafter, as shown in Fig. 4(d), the through hole 21 penetrating the electrode layer 14 and the elastomer layer 16 is formed by irradiation with a laser or the like, then as shown in Fig. 4(e), conductive rubber as the conductive material can be filled in the through hole 21 to form the connector part 22 to obtain the bioelectrode 10.

Although the connector part 22 penetrating the electrode layer 14 and the elastomer layer 16 is formed in the flexible electrode 12 of the bioelectrode 10 shown in Fig. 1 to Fig. 4, the connector part 22 may not be formed as shown in Fig. 5(a). In this case, as shown in Fig. 5(b), it is necessary for the electrode layer 14 to extend to the vicinity of the edge of the elastomer layer 16 so that the external connection terminal 28 can be directly connected to the electrode layer 14.

Further, as shown in Fig. 6, a combination of a conductive nonwoven fabric 30 and a conductive adhesive 32 can be used as the conductive material in the through hole 21. In this case, the total thickness of the conductive nonwoven fabric 30 and the conductive adhesive 32 is preferably 100 µm or less. Furthermore, as shown in Fig. 7, a pad 34 comprising a conductive material and having a larger area than the opening area of the through hole 21 may be formed at the boundary surface of the water-soluble sacrificial layer 20 of the elastomer layer 16. Since the pad 34 is larger than the opening area of the through hole 21, it can be easily connected to an external connection terminal. The pad 34 can be formed by printing a conductive polymer.

Although the bioelectrode 10 comprising the flexible electrode 12 in which the elastomer layer 16 is formed in a larger area than the electrode layer 14 is shown in Fig. 1 to Fig. 7, the bioelectrode may have a flexible electrode in which the elastomer layer 16 and the electrode layer 14 are equivalent. Even with such a flexible electrode, the electrode layer 14 can deform in association with the installation surface of the biological surface so as to adhere to the installation surface, and the accuracy of biological measurement can be improved. However, although the adhesion force between the electrode layer 14 and the installation surface is slightly lower than the adhesion force between the elastomer layer 16 and the installation surface, the bioelectrode having such a flexible electrode can be sufficiently used for disposable use.

### Embodiments

Hereinafter, examples of the present invention will be described in detail, but the scope of the present invention is not limited to these examples.

### Example 1

The relationship between the thickness and the adhesion of the polyurethane sheet to the skin used for the elastomer layer 16 of the bioelectrode 10 according to the present invention was investigated.

### (Preparation of polyurethane sheet)

After 10 mass% of polyvinyl alcohol aqueous solution was applied on a polyethylene terephthalate (PET) sheet with a bar coater, it was heat-treated at 80°C for 1 hour to form a polyvinyl alcohol (PVA) film. Next, a diluted solution in which A and B of Pandex GC manufactured by DIC Co., Ltd. of two-component curable polyurethane were mixed (A:B = 100:17) and diluted with ethyl acetate was applied on the PVA film, heat-treated for 1 hour at 80°C, and cooled to room temperature to obtain a polyurethane sheet of a predetermined thickness. Thereafter, the polyurethane sheet and the PVA film were peeled off from the PET sheet, and the PVA film was dissolved in water to obtain a polyurethane sheet. The thickness of the obtained polyurethane sheet was adjusted by adjusting the polyurethane concentration in the diluted solution diluted with ethyl acetate. The thickness of the obtained polyurethane sheet was verified by measurement using the Dektak XT manufactured by Bruker.

### (Adhesion test of polyurethane sheet to artificial skin)

The degree of adhesion of the obtained polyurethane sheet of a predetermined thickness to the skin was tested. The evaluation was made by using an artificial skin as the skin. As the artificial skin, a Bioskin plate P001-001 manufactured by Beaulax Co., Ltd. was used. In addition, the degree of adhesion to the artificial skin was tested in accordance with the cross-cut method described in JIS K5600-5-6. In the cross-cut method, as shown in Fig. 8(a), after applying a lattice-like notch 46 (2 mm interval) shown in Fig. 8(b) to the urethane sheet 42 that adheres to the artificial skin 40, the adhesive tape 44 having a known peeling strength was attached to the urethane sheet 42. Next, it was pulled off at an angle of 60° in 0.5-1 seconds within 5 minutes of attaching the adhesive tape 44. The peeling state of the lattice-like notch 44 of the pulled adhesive tape 44 was observed and classified into 0 to 5 classifications shown in Fig. 8(c). As determination, 3 or more were considered to be peeled off. Scotch No. 331 manufactured by 3M (peel strength 0.29 N/mm) and Pioran cross Y-03-BL manufactured by Diatex Co., Ltd. (peel strength 0.16 N/mm) were used as the adhesive tapes. The results are shown in Table 1.

### [Table 1]

**Table 1**

| Adhesive tape | Thickness of urethane sheet(µm) | | | | | |
|---|---|---|---|---|---|---|
| | 10 | 5.0 | 1.5 | 1.0 | 0.5 | 0.3 |
| Scotch No. 331 | 5 | 2 | 2 | 0 | 0 | 0 |
| Pioran cross Y-03-BL | 5 | 2 | 2 | 0 | 0 | 0 |
| Determination | NG | OK | OK | OK | OK | OK |

### (Verification of adhesion of polyurethane sheet to artificial skin)

After the obtained polyurethane sheets of thicknesses of 5.0 µm and 0.3 µm were adhered to the artificial skin, electron micrographs (magnification 230) of the cross section were taken. The micrographs are shown in Fig. 9. Fig. 9(a) is the electron micrograph showing the adhesion state between the polyurethane sheet of 5.0 µm in thickness and the artificial skin, and Fig. 9(b) is the electron micrograph showing the adhesion state between the polyurethane sheet of 0.3 µm in thickness and the artificial skin. As is clear from Fig. 9, it was verified that the sheets of both thicknesses adhered to the artificial skin, and in particular, it was verified that the thinner the polyurethane sheet was, the finer it followed the unevenness of the artificial skin.

As is clear from Table 1 and Fig. 9, it was verified that when the thickness of the polyurethane sheet was 5 µm or less, it adhered sufficiently to the artificial skin. In particular, it was verified that it adhered more strongly when it was 1 µm or less.

### Example 2

Next, a PEDOT-PSS sheet was prepared as a conductive polymer used for the electrode layer 14 of the bioelectrode 10, and the relationship between the conductivity and the thickness was investigated.

### (Preparation of PEDOT-PSS sheet)

SP-801 manufactured by Nagase ChemteX Corporation was used as PEDOT-PSS. First, PEDOT-PSS diluted with ethanol was applied with a bar coater onto a polyethylene terephthalate (PET) sheet so that it has a predetermined thickness, and heat treatment was applied at 80°C for 1 hour to obtain the PEDOT-PSS sheet. The thickness was verified by measuring using the Dektak XT manufactured by Bruker as in Example 1.

### (Conductive characteristic test of PEDOT-PSS sheet)

The obtained PEDOT-PSS sheet was tested for conductivity with reference to ANSI/AAMI EC12:2000. The conductivity was determined to be good at 2 kΩ or less at the electrode impedance. The test was performed by processing the obtained PEDOT-PSS sheet to 10 mm × 10 mm and using the multimeter B35T manufactured by Owon. The results are described in Table 2.

### [Table 2]

**Table 2**

| | Thickness of PEDOT-PSS sheet(µm) | | | | |
|---|---|---|---|---|---|
| | 1.2 | 0.5 | 0.4 | 0.2 | 0.1 |
| Electrode impedance(Ω) | 174 | 371 | 798 | 5230 | OL |
| Determination | OK | OK | OK | NG | NG |

As is clear from Table 2, the thicker the PEDOT-PSS sheet was, the more the electrode impedance decreased. It was also verified that the electrode impedance exceeded the specified 2 kΩ at 0.2 µm or less. From this result, the PEDOT-PSS sheet used for the electrode layer 14 is preferably 0.3 µm or more.

### Example 3

The degree of adhesion to the artificial skin was tested by the cross-cut method shown in Fig. 9 for the bioelectrode.

### (Adhesion test to artificial skin using bioelectrode)

First, a polyurethane mixed solution diluted with ethyl acetate was applied onto a piece of water-permeable paper (SP manufactured by Marushige Shiko Co., Ltd.) on which starch was applied in advance as a water-permeable layer so that it had a predetermined thickness as an elastomer layer, then it was heat-treated at 80°C for 1 hour, cooled to room temperature and laminated. Next, PEDOT-PSS diluted with ethanol was applied to the entire surface of the elastomer layer as a conductive layer, and it was cured at 80°C for 1 hour and layered to obtain a bioelectrode. Next, water was supplied to the obtained laminate nonwoven fabric to dissolve the starch layer to remove the nonwoven fabric, and a two-layer laminate of the elastomer layer and the electrode layer was obtained. The electrode layer of this two-layer laminate was adhered to the artificial skin and dried for about 30 minutes, then subjected to an adhesion test to the artificial skin by the cross-cut method shown in Fig. 8. The adhesive tape used for the test was the same as that of Example 1. For comparison, the elastomer layer and the electrode layer alone were tested in the same way. The results are described in Table 3.

### [Table 3]

**Table 3**

| | | | | | | | |
|---|---|---|---|---|---|---|---|
| Thickness of elastomer layer(µm) | | 5.0 | 0.9 | 1.9 | 0.3 | 0.3 | - |
| Thickness of electrode layer(µm) | | - | 3.1 | 2.4 | 1.2 | 1.0 | 0.4 |
| Total thickness(µm) | | - | 4.0 | 4.3 | 1.5 | 1.3 | - |
| Adhesive tape | Scotch No. 331 | 2 | 2 | 2 | 1 | 1 | ND |
| | Pioran cross Y-03-BL | 2 | 1 | 1 | 1 | 1 | ND |
| Determination | | OK | OK | OK | OK | OK | NG |

From the results of Table 3, it was verified that the two-layer laminate of the elastomer layer and the electrode layer was adhered sufficiently to the artificial skin. Especially, it was verified that the thinner the whole thing was, the stronger it adhered to the artificial skin. In addition, the strength of the electrode layer alone was weak, and it was destroyed on the artificial skin, and tests could not be performed. Therefore, it was determined that it was preferably used in conjunction with the elastomer layer, and more preferably, the elastomer layer was larger than the electrode layer in order to protect the electrode layer, and thereby obtaining the good adhesion and strength stability of the two-layer laminate to the artificial skin.

From Table 1 to Table 3 and Fig. 9, preferably, the thickness of the elastomer layer is 5 µm or less, the thickness of the electrode layer is 0.3 µm or more, and the total thickness of both is 5.3 µm or less to improve the adhesion of the two-layer laminate to the artificial skin. Furthermore, when the area of the elastomer layer is larger than that of the electrode layer, the degree of adhesion of the two-layer laminate to the artificial skin can be improved.

### Example 4

For the purpose of improving the durability of the two-layer laminate, surface treatment was performed on the elastomer layer, and the effect was verified.

### (Conductivity characteristic test by surface treatment)

The polyurethane with a thickness of 0.3 µm was formed on a piece of water-permeable paper having a starch layer as an elastomer layer as in Example 3. Next, the treatment shown from the left to the right of Table 4 below was sequentially performed on the entire surface of the elastomer layer.

### [Table 4]

**Table 4**

| Treatment procedure | Corona discharge step | VSCA treatment step | Heat treatment step | Corona discharge step | DASCA treatment step | Electrode layer application step |
|---|---|---|---|---|---|---|
| Condition 1 (No treatment) | - | - | - | - | - | 1 |
| Condition 2 (Corona treatment) | 1 | - | - | - | - | 2 |
| Condition 3 (VSCA treatment) | 1 | 2 | 3 | 4 | x | 5 |
| Condition 4 (DASCA treatment) | 1 | - | - | - | 2 | 3 |

In the "corona discharge step" in Table 4, discharge at 17 kV from a distance of 1 mm in air was performed three times at a speed of 70 mm/s. In the "VSCA treatment step", an ethanol solution of 0.1 mass% of alkoxy siloxane containing a plurality of vinyl groups (VMM-010 manufactured by AZmax Co., Ltd.) was applied as a vinyl group silane coupling agent. In the "heat treatment step", heating was performed at 80°C for 10 minutes. In the "DASCA treatment step", an ethanol solution of 0.1 mass% of 3-(2-aminoethyl)aminopropyltrimethoxysilane (OFS-6020 manufactured by Dow Toray Co., Ltd.) was applied as a diamino group silane coupling agent.

PEDOT-PSS diluted with ethanol was applied as an electrode layer on the elastomer layer subjected to each treatment as "an electrode layer application step", and it was cured at 80°C for 1 hour to laminate an electrode layer of 0.6 µm with an area of 10 mm × 30 mm. The electrical resistance between 10 mm of the obtained electrode layer was measured. The result is shown in Fig. 10.

As is clear from Fig. 10, the electrical resistance value of the layer subjected to "DASCA treatment" to the elastomer layer was the lowest. It is presumed that the wettability of the elastomer layer was improved by subjecting the elastomer layer to "DASCA treatment" and the ethanol solution of PEDOT-PSS was uniformly applied. In any treatment subjected to the polyurethane shown in Table 4, the electrical resistance value of the obtained electrode layer can be used as an electrode.

### (Durability improvement test by surface treatment)

After each treatment was applied to the elastomer layer, the tensile durability was examined for the two-layer structure in which the electrode layer was formed. As shown in Fig. 11(a) and (b), two wires 52 with a width of 2 mm were printed at intervals of 5 mm with silver paste on the upper surface of the artificial skin 40. The 9 mm × 9 mm two-layer structure 11 in which the electrode layer 14a was laminated on the entire surface of the elastomer layer 16a was attached onto the wires 52 and 52 so that the electrode layer 14a and the wires 52 and 52 were adhered to each other and dried at room temperature. Next, as shown in Fig. 11(b), the artificial skin 40 was pulled a predetermined distance in the arrow direction, and immediately returned to measure the electrical resistance value (R) between the wires 52 and 52 to obtain the ratio (R/RO) to the electrical resistance value (RO) between the wires 52 and 52 before pulling. The result is shown in Fig. 12.

As is clear from Fig. 12, it was verified that the two-layer structure obtained by "DASCA treatment" had a small R/RO ratio and excellent tensile durability even if it was expanded and contracted. In addition, it can be used repeatedly as its durability was improved. It is presumed that since the PSS (polystyrene sulfonate) of PEDOT-PSS forming the electrode layer 14a was acidic, it was attracted to an alkaline amine group.

### Example 5

A bioelectrode was prepared, compared with commercially available gel electrodes, muscle potential measurements, and cardiac potential measurements were performed.

### (Preparation of the bioelectrode 10)

A water-soluble sacrificial layer 20 made of starch was formed on one surface side of the water-permeable layer 18 consisting of a nonwoven fabric made of cellulose. A diluted solution in which A and B of Pandex GC manufactured by DIC Co., Ltd. of two-component curable polyurethane were mixed (A: B = 100: 17) and diluted with ethyl acetate was applied on the entire surface of the water-soluble sacrificial layer 20, heat-treated at 80°C for 1 hour, and cooled to room temperature to form the elastomer layer 16 consisting of polyurethane of 0.3 µm. Next, the elastomer layer 16 was covered with a PET sheet with a predetermined open area as a mask, and after printing a diluted solution in which PEDOT-PSS of SP-801 manufactured by Nagase ChemteX Corporation was diluted with ethanol, it was cured at 80°C for 1 hour to form the electrode layer 14 of 0.6 µm in thickness having a predetermined area. The area ratio between the elastomer layer 16 and the electrode layer 14 (elastomer layer 16: electrode layer 14) constituting the flexible electrode 12 was 6:1.

Furthermore, after forming a through hole of 5 mm in diameter penetrating the electrode layer 14 and the elastomer layer 16, conductive silicone in which silver and aluminum fillers are blended in the silicone adhesive was filled in the through hole, and the heat treatment was applied at 80°C for 1 hour to form the connector part 22 to obtain the bioelectrode 10 shown in Fig. 1.

### (Measurement of muscle potential and comparison with gel electrodes)

After adhering the conductive layer side of the obtained bioelectrode 10 to the skin parts shown with marks in a circle on the left arm shown in Fig. 13(a), water was supplied to the water-permeable layer 18 to dissolve the water-soluble sacrificial layer 20 to remove the water-permeable layer 18 to merely leave the flexible electrode 12. Next, an external terminal of the electromyograph was installed to the exposed end of the connector part 22 exposed on the surface of the elastomer layer 16 of the flexible electrode 12, and the muscle potential was measured with a grip strength meter held in the left hand when held so as to indicate a predetermined grip force. Measurements were made using Bitalino and data was stored using software Opensignals. The result is shown in Fig. 13(b). Further, the measurement result of the muscle potential similarly performed using the disposable electrode F Vitrode of the gel electrode manufactured by Nihon Kohden Co., Ltd. as a reference electrode is also shown in Fig. 13(c). Furthermore, the signal noise ratio between the flexible electrode 12 and the reference electrode (Max value during activity (S)/Max value at rest (N)) is shown in Table 5 below.

### [Table 5]

**Table 5**

| | | | | |
|---|---|---|---|---|
| Grip strength | 5kg | 10kg | 15kg | 20kg |
| S/N of flexible electrode12 | 0.39 | 0.60 | 1.02 | 1.22 |
| S/N of reference electrode | 0.13 | 0.24 | 0.41 | 0.52 |

As is clear from Fig. 13 and Table 5, the flexible electrode 12 was able to obtain the same signal as the reference electrode.

### (Correspondence between fist movement and arm muscle potential)

After adhering the conductive layer side of the obtained bioelectrode 10 to the skin of the arm as shown in Fig. 14(a), water is supplied to the water-permeable layer 18 to dissolve the water-soluble sacrificial layer 20, in order to remove the water-permeable layer 18 to merely leave the flexible electrode 12. Next, as shown in Fig. 14(a), the external terminals 52 and 52 of the electromyograph were connected to the exposed end of the connector part 22 exposed to the surface of the elastomer layer 16 of the flexible electrode 12 to measure the muscle potential.

As shown in Fig. 14(a), it can be seen that the muscle potential of the arm changes correspondingly as shown in Fig. 14(b) when the fist of the arm equipped with the flexible electrode 12 is clenched or opened.

### (Measurement of cardiac potential)

The cardiac potential was measured using the obtained flexible electrode 12 of the bioelectrode 10. The state in which the flexible electrode 12 is installed is shown in Figs. 15(a) and (b) with marks in a circle. The positive electrode P was installed to the palm side wrist of the left hand as shown in Fig. 15(a), and the negative electrode N was installed to the palm side wrist of the right hand as shown in Fig. 15(a). Further, the reference electrode R was installed to the back side wrist of the left hand as shown in Fig. 15(b). The cardiac potential was measured using Bitalino and the data was stored using the software Opensignals. The result is shown in Fig. 16(a). Further, the measurement result of the cardiac potential similarly performed using the disposable electrode F Vitrode of the gel electrode manufactured by Nihon Kohden Co., Ltd. as a reference electrode is also shown in Fig. 16(b). The PQ time and QT time shown in Fig. 16(c) were measured using the measured electrocardiogram. The PQ time is the transmission time from the atrium to the ventricle, and the QT time is the time from ventricular excitement to the end. The results are shown in Table 6.

### [Table 6]

**Table 6**

| | Flexible electrode12 | Reference electrode | General average value |
|---|---|---|---|
| PQ Time(sec) | 0.151 | 0.137 | 0.12-0.20 |
| QT Time(sec) | 0.411 | 0.397 | 0.36-0.44 |

As is clear from Figs. 16(a) to (c) and Table 6, the flexible electrode 12 and the reference electrode showed the same results.

### Industrial Applicability

According to the bioelectrode according to the present invention, the accuracy of biological measurement can be improved, and it can contribute to the prevention of diseases and the improvement of healthy life expectancy.

### Explanations of Letters or Numerals

10: bioelectrode, 11: two-layer structure, 12: flexible electrode, 14 and 14a: electrode layers, 16 and 16a: elastomer layers, 18: water-permeable layer, 20: water-soluble sacrificial layer, 21: through hole, 22: connector part, 24: installation surface, 26: water, 28: external connection terminal, 30: conductive nonwoven fabric, 32: conductive adhesive, 34: pad, 40: artificial skin, 42: urethane sheet, 44: adhesive tape, 46: notch, 52: a wire, P: positive electrode, N: negative electrode, R: reference electrode.

## Claims

1. A bioelectrode in which a flexible electrode that is to directly contact a biological surface is formed from an electrode layer that comprises a conductive polymer and deforms in association with an installation surface of the biological surface so as to adhere to the installation surface, and an elastomer layer that is layered on one surface side of the electrode layer and deforms in association with the installation surface and the electrode layer,
wherein the flexible electrode is bonded to a water-permeable layer that serves as a support via a water-soluble sacrificial layer that comprises a water-soluble material.

2. The bioelectrode according to claim 1, wherein the elastomer layer has a larger area than the electrode layer so that the exposed surface from the electrode layer adheres to the installation surface.

3. The bioelectrode according to claim 1 or claim 2, wherein the thickness of the elastomer layer is 5 µm or less, the thickness of the electrode layer is 0.3 µm or more, and the total thickness of the elastomer layer and the electrode layer is 5.3 µm or less.

4. The bioelectrode according to any one of claims 1 to 3, wherein the conductive polymer is poly(3,4-ethylenedioxythiophene)-polystyrene sulfonate.

5. The bioelectrode according to any one of claims 1 to 4, wherein the elastomer layer is formed of polydimethylsiloxane, polyurethane or styrene-butadiene thermoplastic elastomers.

6. The bioelectrode according to any one of claims 1 to 5, wherein the water-permeable layer is formed of a nonwoven fabric made of cellulose or resin, or a sponge or mesh made of resin.

7. The bioelectrode according to any one of claims 1 to 6, wherein the water-soluble sacrificial layer is formed of starch, polyvinyl alcohol, polyacrylic acid or polyethylene glycol.

8. The bioelectrode according to any one of claims 1 to 7, wherein a conductive material is filled in a through hole penetrating through the elastomer layer and the electrode layer, and a conductive passage that electrically connects the electrode layer to an external connection electrode installed to the other surface side of the elastomer layer is formed.

9. A method for producing a bioelectrode including a flexible electrode directly contacting a biological surface comprising:
laminating a water-soluble sacrificial layer comprising a water-soluble material on one surface side of a water-permeable layer serving as a support layer of the flexible electrode;
and then laminating sequentially an elastomer layer and an electrode layer on the water-soluble sacrificial layer;
wherein the electrode layer comprising a conductive polymer and deforming in association with an installation surface of the biological surface so as to adhere to the installation surface, and the elastomer layer layered on one surface side of the electrode layer and deforming in association with the installation surface and the electrode layer constitute the flexible electrode.

10. The method for producing a bioelectrode according to claim 9, wherein the elastomer layer is formed in a larger area than the electrode layer so that the exposed surface from the electrode layer adheres to the installation surface.

11. The method for producing a bioelectrode according to claim 9 or claim 10, wherein the elastomer layer having a thickness of 5 µm or less is formed and the electrode layer having a thickness of 0.3 µm or more is formed so that the total thickness of the elastomer layer and the electrode layer is 5.3 µm or less.

12. The method for producing a bioelectrode according to any one of claims 9 to 11, wherein poly(3,4-ethylenedioxythiophene)-polystyrene sulfonate is used as the conductive polymer.

13. The method for producing a bioelectrode according to any one of claims 9 to 12, wherein the elastomer layer is formed of polydimethylsiloxane, polyurethane or styrene-butadiene thermoplastic elastomers.

14. The method for producing a bioelectrode according to any one of claims 9 to 13, wherein the water-permeable layer is formed of a nonwoven fabric made of cellulose or resin, or a sponge or mesh made of resin.

15. The method for producing a bioelectrode according to any one of claims 9 to 14, wherein the water-soluble sacrificial layer is formed of starch, polyvinyl alcohol, polyacrylic acid or polyethylene glycol.

16. The method for producing a bioelectrode according to any one of claims 9 to 15, wherein a conductive material is filled in a through hole that penetrates the elastomer layer and the electrode layer, and a conductive passage that electrically connects the electrode layer to an external connection electrode installed to the other surface side of the elastomer layer is formed.

17. A method for installing a bioelectrode, the method for installing a bioelectrode according to any one of claims 1 to 8 to the installation surface of the biological surface comprising:
bringing an entire surface of the one surface side on which the electrode layer of the flexible electrode constituting the bioelectrode is formed into contact with the installation surface; adhering the electrode layer to the installation surface while the elastomer layer and the electrode layer deform in association with the installation surface;
then supplying water to the water-permeable layer to dissolve a water-soluble material forming the water-soluble sacrificial layer with water penetrated through the water-permeable layer;
and peeling the water-permeable layer off from the flexible electrode.

18. The method for installing a bioelectrode according to claim 17, wherein the bioelectrode according to claim 2 in which the elastomer layer is formed in a larger area than the electrode layer is used as the bioelectrode, and while the elastomer layer and the electrode layer deform in association with the installation surface, and the electrode layer is adhered to the installation surface, an exposed surface of the elastomer layer from the electrode layer is adhered to the installation surface.
